# EUROPEAN PATENT APPLICATION

(11) **EP 2 801 328 A1**
(43) Date of publication of application: **12.11.2014**
(21) Application number: 13305591.3
(22) Date of filing: 06.05.2013
(51) Int. Cl.: A61B 17/70

(54) **Intervertebral device**

(71) Applicant: ZIMMER SPINE, 33000 Bordeaux (FR)
(72) Inventor: Ichelmann, Bruno, 87100 Limoges (FR)
(74) Representative: Venner Shipley LLP

(57) **Abstract**

An intervertebral device (1) to be placed between two spinous processes of two adjacent vertebrae, comprising a spacer (10) having two opposite grooves (14A, 14B). Each groove is configured to engage a spinous process. At least one porous layer (51A, 52A, 53A, 51B, 52B, 53B) is fixed onto an inner face of each groove. This porous layer is adapted to interface with the spinous process by allowing bone growth in the porous layer. One or several elongated members (30A, 30B) are adapted to surround the spinous processes for maintaining the spinous processes engaged into the grooves (14A, 14B).

## Description

### TECHNICAL FIELD

The present disclosure relates to an intervertebral device to be placed between two spinous processes of two adjacent vertebrae. Such a device is sometimes called an "interspinous device" or an "interspinous implant". It is used for holding two vertebrae in a desired relative position.

### BACKGROUND

The spine is formed of superposed vertebrae, from the lumbar vertebrae to the cervical vertebrae, each having an anterior part, which is the vertebral body, and a posterior part, which is the vertebral arch (or neural arch), the anterior and posterior parts enclosing the vertebral foramen. Each vertebral arch is formed by a pair of pedicles and a pair of laminae, and has transverse processes and/or a spinous process (or neural spine) projecting therefrom. The transverse and spinous processes project opposite to the vertebral foramen.

An intervertebral disc lies between each pair of adjacent vertebrae, i.e. between the vertebral bodies of these vertebrae. Each disc forms a cartilaginous joint to hold the two adjacent vertebrae together while allowing relative movements between these vertebrae. When an intervertebral disk is damaged (e.g. worn out or degenerated), it becomes unable to prevent excessive relative movements between the two vertebrae contiguous to the disk, during flexion (forward movement) or extension (backward movement) of the spine.

An abnormal excessive relative movement between two adjacent vertebrae, whether due to a damaged disk or any other reasons, may cause discomfort and/or pain to the patient.

As a remedy to this problem, it is known to place an intervertebral device between the spinous processes of the two adjacent vertebrae. Such a device limits the extent to which the posterior parts of the two vertebrae can move with respect to each other when the spine is extended and/or flexed. In other words, the device provides stabilization between the vertebrae. This stabilization may be more or less "dynamic" depending on the amount of relative movement allowed between the vertebrae. The less the amount of relative movement allowed, the less dynamic or the more "static" the stabilization.

Known examples of intervertebral devices are disclosed, for instance, in US patents No. 7087083, 7163558 and 7520887. Those devices provide dynamic stabilization.

Another example of an intervertebral device is disclosed in US patent No. 5645599. This device provides more static stabilization than the above-mentioned ones. However, implantation of this device can be traumatic and invasive because pins or crimped spikes are inserted into the spinous processes of the vertebrae.

Further, so-called fusion is known in which vertebrae are fixedly connected to each other. It is known, for example, to use so-called cages which are inserted between the vertebrae, as disclosed in European patent No. 1299054. Such surgery is much more invasive that an interspinous surgery, as commonly e.g. the disc needs to be removed and the surgeon needs to work around the spinal nerves. It is moreover known to affect fusion by means of rigid posterior rod systems which are attached to either pedicles or laminae of the vertebrae by means of hooks or screws. Such systems require access to further structures of the vertebrae themselves, compared to access to the spinous processes only. Such systems are known for example from European patent No. 348272.

### GENERAL PRESENTATION

According to one embodiment, there is provided an intervertebral device to be placed between two spinous processes of two vertebrae. The intervertebral device comprises a spacer having two opposite or opposing grooves, each groove being configured to engage a spinous process, wherein at least one porous layer is provided on an inner face of each groove, this porous layer being adapted to interface with the spinous process by allowing bone growth in the porous layer.

Such a device allows, besides other advantages, rigid or largely rigid connection between two vertebrae by a surgery which is as little invasive as dynamic stabilization by means of an interspinous spacer.

The porous layer may be made of material(s) commonly known as "bone-ingrowth material(s)" configured to allow bone ingrowth into the pores of the porous material. It is made of one or several porous materials, such as porous metals and/or porous ceramics, for example. The porous layer may comprise sub-layers of different materials and/or properties (e.g. different porosities). For example, the porous layer may be formed using Trabecular Metal™ technology available from Zimmer, Inc., of Warsaw, Indiana. Trabecular Metal™ is a trademark of Zimmer Technology, Inc. Such a material may be formed from a reticulated vitreous carbon foam substrate which is infiltrated and coated with a biocompatible metal, such as tantalum, etc., by a chemical vapor deposition ("CVD") process in the manner disclosed in detail in U.S. Patent No. 5282861, issued February 1, 1994, entitled "open cell tantalum structures for cancellous bone implants and cell and tissue receptors", the entirety of which is included herein by reference, and in Levine, B.R., et al., "Experimental and Clinical Performance of Porous Tantalum in Orthopedic Surgery", Biomaterials 27 (2006) 4671-4681. Further methods of producing porous metal structures, such as Trabecular Metal™ structures, are provided in WO 2012/065729. In addition to tantalum, other metals such as niobium or alloys of tantalum and niobium with one another or with other metals may also be used. Bone-ingrowth materials other than Trabecular Metal™ may also be used. For instance, Trabecular Titanium™ available from Lima Corporate may be used.

The porous layers may be fixed onto the inner faces of the grooves by pressing, friction, swaging, clamping, gluing, interlocking or any other appropriate fixing technique. For instance, the porous layers may be fitted into recesses of complementary shape provided in the spacer.

After bone growth is achieved into the porous layer, the two spinous processes are directly bound to the porous layers which are fixed to the spacer. Accordingly, the spinous processes are held together via the spacer.

The spacer and, more particularly, the central part of it may be slightly deformable in some instances. When the central part is slightly deformable, it may allow a limited amount of relative movement between the vertebrae. In other instances, the spacer may generally not be deformable, i.e., rigid. When the central part is not deformable, the amount of relative movement allowed between the vertebrae is zero: the intervertebral device provides static stabilization. Such a device providing static stabilization may provide a desired effect for spinal fusion surgery. Spinal fusion is a surgical technique used to permanently join adjacent vertebrae by creating a motionless junction between the vertebrae (i.e. the vertebrae are immobilized with respect to each other).

The spacer may be made of any desired material, such as polymer and/or metallic materials. For example, in some instances the spacer may be formed of polyetheretherketone (PEEK) or titanium alloy.

In some embodiments, the intervertebral device may include one or more securement features configured to engage the spinous processes in order to secure the intervertebral device to the spinous processes prior to establishing bone ingrowth into the pores of the porous layer.

In certain embodiments, the intervertebral device comprises at least one elongated member adapted to surround the spinous processes for maintaining the spinous processes engaged into the grooves. Thus, the elongated member allows the spinous processes to be held in position with respect to the spacer and the porous layers, which fosters bone growth in the porous layer. Alternatively or cumulatively,, the intervertebral device may include pins, spikes, or other fasteners to initially secure the intervertebral device to the spinous processes. However, passing the elongated member(s) around the spinous processes is less invasive than passing pins or spikes through the spinous processes.

The elongated member may be a tie having a band shape, a cord shape or other shapes. For example, it may be made by weaving or as a cable. It may be made from a polymeric material such as, for example, polyester, polyethylene (for example, polyethylene terephthalate, i.e. PET), polyetheretherketone (PEEK).

A single elongated member may be passed around the two spinous processes or one elongated member may be passed around each spinous process. The elongated member or members may form a loop around each spinous process, this loop being tightened against the spinous process, for instance by pulling on the end(s) of the elongated member. The elongated member may be fastened to the spacer by any appropriate fastening technique and/or system, such as knotting, sewing, a self-locking system, a clip-fixing system, a clamping system, or a combination thereof.

In certain embodiments, each groove is defined between two branches and a porous layer is provided at least on the inner face of the distal part of each branch. Thus, after bone growth is achieved into the porous layers, the two branches are joined via the spinous process fitted in the groove. Alternatively or cumulatively, a porous layer may be provided at a base or bottom of each groove. Thus, after bone growth in the porous layers, the two spinous processes fitted in the grooves are joined via the spacer.

The porous layer may comprise or be made from a material consisting of a web of interconnected rods. The rods may leave interconnected voids between them. The rods may have a metallic surface and/or be made from a metal or alloy. The metallic surface may be made from tantalum, niobium, titanium or an alloy of any two or more thereof. The rods may consist of metal coated pyrolized polymeric material.

In certain embodiments, the porous layer may have a highly open porosity that imitates the morphology of cancellous bone. For instance, the above-mentioned rods may form a three dimensional structure, said structure being, in particular, a trabecular structure as found in cancellous bone.

In certain embodiments, each groove is defined between two branches extending from a central part of the spacer and at least one branch is deformable toward the other branch. The two branches may be deformable toward each other. Then, the branches may be deformed toward each other to clamp the spinous process between them. In some embodiments, at least a portion of each branch, in particular the distal part of the branch (i.e., the end of the branch furthest from the central part of the spacer), is deformable. For example, tensioning the elongated member(s) may draw the distal parts of the branches toward one another and bring the porous layer into contact with the spinous processes. When a porous layer is provided on the inner face of the distal parts, the contact between the porous layers and the spinous process is thus improved.

In certain embodiments, the elongated member passes along an outer face of each branch. When the branches are deformable, they may thus be deformed and brought into closer contact with the spinous process by tightening the elongated member around the branches and the spinous process.

In certain embodiments, an insert extends within the spacer, between the two opposite grooves, the insert having two opposite ends, each end defining a porous layer at the bottom of each groove. Accordingly, bone growths from the two spinous processes take place in the same insert, thus creating a rigid bone fusion between the adjacent spinous processes.

According to another aspect of the present disclosure, there is provided a method for stabilizing at least two adjacent vertebrae, comprising the steps of providing an intervertebral device according to the present disclosure and impeding relative movement between the vertebrae by placing the intervertebral device between two spinous processes of the vertebrae.

Such a method is used, in particular, in spinal fusion surgery.

This method has the advantages derived from using an intervertebral device according to the present disclosure.

Other features and advantages of the invention will become apparent from the following detailed description, taken in conjunction with the accompanying drawings, which illustrate, by way of example, the principles of the invention.

### BRIEF DESCRIPTION OF THE DRAWINGS

In the drawings, like reference signs generally refer to the same parts throughout the different views.
FIG 1 is a perspective view of an example of an intervertebral spacer.
FIG 2 is a perspective view of another example of an intervertebral spacer.
FIG 3 is a front view of the intervertebral device of FIG 1 in position between two spinous processes.

### DETAILED DESCRIPTION OF EXAMPLE(S)

In the following detailed description, it is referred to the accompanying drawings showing examples of intervertebral devices. It is intended that these examples be considered as illustrative only, the scope of the invention not being limited to these examples.

To avoid details not necessary to enable those skilled in the art to practice the invention, the present description may omit certain information known to those skilled in the art.

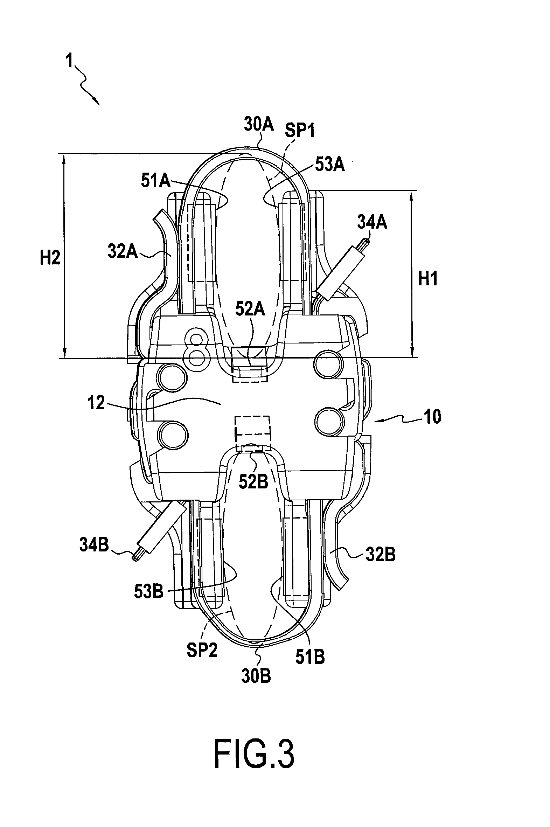

The appended drawings show examples of intervertebral devices 1. Those devices are adapted to be placed between two adjacent spinous processes SP1, SP2 of two vertebrae, as illustrated in FIG 3.

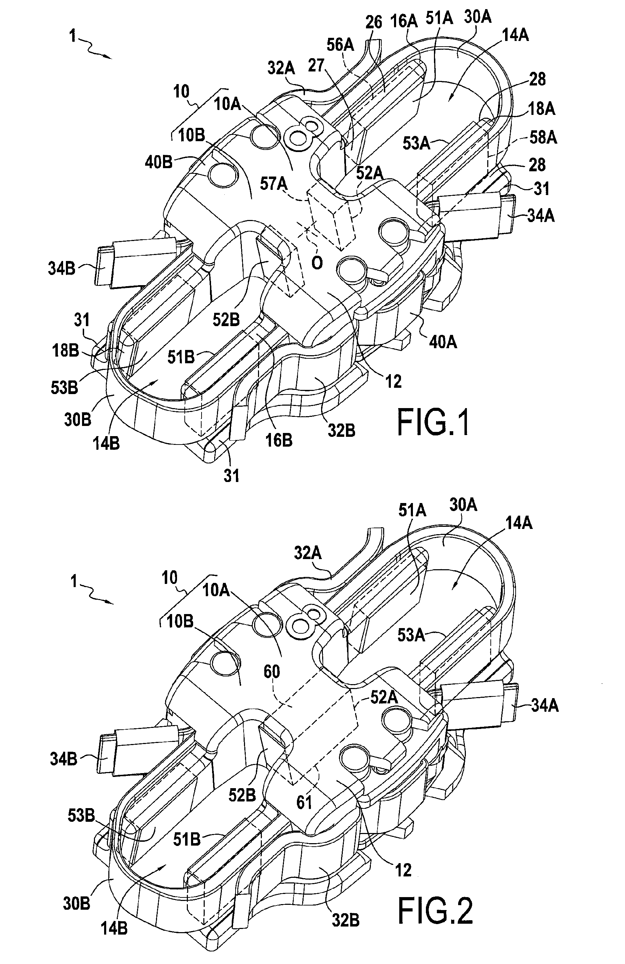

In the example of FIGS 1 and 3, the intervertebral device 1 comprises a spacer 10 having two opposite grooves 14A, 14B adapted to receive the two spinous processes SP1, SP2, respectively, therein and engage the two spinous processes SP1, SP2, respectively. Each groove 14A (14B) is defined between two legs or branches 16A, 18A (16B, 18B). The branches 16A, 18A (16B, 18B) may be high enough (see their height H1 in FIG 3) to cover more than one-half (1/2) of the height H2 of the spinous process SP1 (SP2) concerned, in particular, more than two-thirds (2/3) of the height H2 and, especially, about or more than three-quarters (3/4) of the height H2, the heights H1 and H2 being measured along the central axis of the groove. In other words, the legs or branches 16A, 18A (16B, 18B) may have a length sufficient to extend along 50% or more, 66% or more, or 75% or more of the height H2 of the spinous process SP1 (SP2), in some instances. Since heights H2 of the spinous processes may vary from one patient to another, kits of several intervertebral devices 1 with various groove heights H1 and/or branch lengths may be provided.

In the illustrated example, the device 1 is quasi-symmetrical with respect to a center point 0 (see FIG 1) located in the middle of the central part 12 of the spacer 10. Thus, the spacer 10 comprises two quasi-symmetrical halves 10A, 10B and parts or elements of these two halves having identical or analogous functions, are identified by the same reference number followed by "A" for one part and "B" for the other part. It is noted that the device 1 need not be symmetrical, and in such instances parts or elements of the device 1 associated with, interacting with, or directed toward the first spinous process SP1 may be identified with a reference number followed by "A" while similar parts or elements of the device 1 associated with, interacting with, or directed toward the second spinous process SP2 may be identified with a reference number followed by "B".

The device 1 may comprise two elongated members (see FIG 2), each being formed by a tie 30A, 30B having a band shape, and each tie 30A (30B) having a first, proximal end 32A (32B) and a second, distal end 34A (34B). The proximal end 32A (32B) of each tie 30A (30B) may be permanently fastened to the spacer 10. For instance, the proximal end 32A (32B) may be sewed into a loop, this loop surrounding a part of the spacer 10. The distal end 34A (34B) of each tie 30A (30B) may be passed through a fastening system 40A (40B). Here, the fastening system 40A (40B) is a self-locking system analogous to the one described in French patent application No. FR2897771 and is clipped onto the spacer 10.

Each tie 30A (30B) may extend across the groove 14A (14B) from one branch 16A (18A) to the other branch 16B (18B) to capture the spinous process SP1 (SP2) in the groove 14A (14B). For example, from its proximal end 32A (32B) to its distal end 34A (34B), each tie 30A (30B) may pass along the outer face 26 of one branch 16A (18A), over the groove 14A (14B), along the outer face 29 of the other branch 16B (18B), and through the fastening system 40A (40B).

The branches 16A, 18A (16B, 18B) may comprise a guide for guiding the tie 30A along their outer face. In the illustrated example, each branch 16A, 18A (16B, 18B) has a rib 31 extending longitudinally along the outer face of the branch, from the spacer 10 to the distal end of the branch. The rib 31 may be located on one lateral edge of the branch. For instance, on the lateral edge close to the vertebral foramen when the spacer 10 is implanted. The tie 30A may abut against or extend along the rib 31 and, thus, is guided and prevented from sliding off the branch. Alternatively, each branch 16A, 18A may comprise two ribs 31, one on each of its lateral edges, with the tie 30A extending therebetween.

As illustrated in FIG 3, each tie 30A (30B) is tightened around the spinous processes SP1 (SP2), by pulling on the free, distal end 34A (34B) of the tie 30A (30B). Thus, the spinous processes SP1, SP2 may be retained by the ties 30A, 30B in the grooves 14A, 14B (see FIG 3). In addition, when the distal parts of the branch 16A, 18A, 16B, 18B are deformable, these distal parts may move towards each other when the tie 30A, 30B is tightened, thereby clamping the spinous processes SP1, SP2 between them.

As illustrated, porous layers 51A, 52A, 53A (51B, 52B, 53B) are fixed onto the inner face of each groove 14A (14B), or otherwise positioned to face and contact the spinous process SP1 (SP2) retained in the groove 14A (14B). In particular, two porous layers 51A, 53A may be fixed on the inner faces 27, 29 of the branches 16A, 18A of the groove 14A and one porous layer 52A may be fixed on the bottom of the groove 14A. Each porous layer 51A, 52A, 53A is adapted to interface with the spinous process SP1 by allowing bone growth from the spinous process SP1 into pores of the porous layer. Each porous layer may be made of one or several bone-ingrowth materials such as, for instance, Trabecular Metal™. The porous layers 51A, 52A, 53A may be respectively fitted into recesses 56A, 57A, 58A of complementary shape provided in the spacer 10. In some instances, the porous layers 51A, 52A, 53A (51B, 52B, 53B) may be an integrally formed, continuous layer extending around the groove 14A (14B), or the porous layers 51A, 52A, 53A (51B, 52B, 53B) may be separate, discrete, discontinuous segments positioned around the groove 14A (14B).

Another example of an intervertebral device is shown in FIG 2. This example differs from that of FIGS 1 and 3 in that the porous layers 52A, 52B located at the bottom of each groove 14A, 14B, belong to one insert 60 extending within the spacer 10, between the grooves 14A, 14B. In particular, the porous layer 52A, 52B are defined by two opposite ends of the insert 60. The insert 60 may be partially (i.e. at least at its ends extending into the groove 14A (14B) for engagement with the spinous process) or completely porous. It may be partially or completely made of one or several bone-ingrowth materials such as, for instance, Trabecular Metal™. The insert 60 may be fitted into a through hole 61 of complementary shape provided in the spacer 10, or the spacer 10 may be molded or otherwise formed around the insert 60, for example.

In general, each porous layer 51A, 52A, 53A (51B, 52B, 53B) may protrude from the inner face of the groove 14A (14B), which allows a closer contact between the porous layer and the spinous process SP1 (SP2) and, therefore, fosters bone growth in the porous layer.

Now that the structure of the intervertebral device 1 has been described, the operation of the device 1 is quickly described below.

In some instances the proximal portions 32A, 32B and/or distal portions 34A, 34B of the ties 30A, 30B may be pre-assembled to the spacer 10 before packaging the device. However, in other instances the ties 30A, 30B may be intraoperatively assembled with the spacer 10. The device 1 may be packaged in a sterilized container (e.g. a bag) under an aseptic condition. The packaged device 1 may be then stored and/or delivered to a physician, or other operative.

In operative conditions, the intervertebral device 1 may be used as follows:
- a posterior access to spinous processes SP1, SP2 is created through an incision in the patient;
- the device 1 is inserted between the spinous processes SP1, SP2;
- the ties 30A, 30B are passed around the spinous processes SP1, SP2, respectively, and the distal ends 34A, 34B of said ties are passed, through the fastening systems 40A, 40B, respectively;
- tension is applied to the ties 30A, 30B by pulling, respectively, on their distal ends 34A, 34B, so as to tighten the ties 30A, 30B on the spinous processes SP1, SP2, thereby holding the spinous processes SP1, SP2 in the grooves 14A, 14B.Thus, once the ties 30A, 30B are tightened, the spinous processes SP1, SP2 are held in a stable position with respect to the spacer 10 with the spinous processes SP1, SP2 contacting or pressing against the porous layers 51A-53A, 51B-53B. The apposition of the spinous processes SP1, SP2 with the porous layers fosters subsequent bone growth from the spinous processes SP1, SP2 into the porous layers 51A-53A, 51B-53B. It is noted that bone bleeding improves the bone growth from the spinous processes into the porous layers. Accordingly, the spinous processes may be abraded before putting the spacer 10 in place.

Once bone has grown into the pores of the porous layers 51A-53A, 51B-53B, the spinous processes SP1, SP2 are directly bound or fused to the porous layers 51A-53A, 51B-53B which are fixed to the spacer 10. Accordingly, the spinous processes SP1, SP2 are secured together via the spacer 10 and stabilization is provided between the vertebrae having the spinous processes SP1, SP2.

"Comprises/comprising" when used herein is taken to specify the presence of stated features but does not preclude the presence or addition of one or more other features.

The above description is given by way of example, and not limitation. Given the above disclosure, one skilled in the art could devise variations that are within the scope of the invention. Further, the various features of the embodiments or examples disclosed herein can be used alone or in varying combinations with each other, and are not intended to be limited to the specific combinations disclosed herein.

## Claims

1. An intervertebral device to be placed between two spinous processes of two adjacent vertebrae, comprising a spacer (10) having two opposite grooves (14A, 14B), each groove being configured to engage a spinous process (SP1, SP2), wherein at least one porous layer (51A, 52A, 53A, 51B, 52B, 53B) is provided on an inner face of each groove, this porous layer being adapted to interface with the spinous process by allowing bone growth in the porous layer.

2. The intervertebral device of claim 1, comprising at least one elongated member (30A, 30B) adapted to surround the spinous processes (SP1, SP2) for maintaining the spinous processes engaged into the grooves (14A, 14B).

3. The intervertebral device of claim 1 or 2, wherein each groove (14A, 14B) is defined between two branches (16A, 18A, 16B, 18B) extending from a central part of the spacer (10) and wherein a porous layer (51A, 53A, 51B, 53B) is provided on the inner face of each branch.

4. The intervertebral device of any one of claims 1 to 3, wherein each groove (14A, 14B) is defined between two branches (16A, 18A, 16B, 18B) extending from a central part of the spacer and wherein at least one branch is deformable toward the other branch.

5. The intervertebral device of claim 3 or 4, wherein the elongated member (30A, 30B) passes along an outer face of each branch.

6. The intervertebral device of any one of claims 1 to 5, wherein a porous layer (52A, 52B) is provided at a base of each groove (14A, 14B).

7. The intervertebral device of any one of claims 1 to 6, wherein an insert (60) extends within the spacer (10), between the two opposite grooves (14A, 14B), the insert (60) having two opposite ends, each end defining a porous layer (52A, 52B) at a base of each groove.

8. The intervertebral device of any one of claims 1 to 7, wherein the porous layer (51A, 52A, 53A, 51B, 52B, 53B) comprises porous metal.

9. The intervertebral device of any one of claims 1 to 8, wherein the porous layer (51A, 52A, 53A, 51B, 52B, 53B) is made from a material consisting of a web of interconnected rods.

10. The intervertebral device of claim 9, wherein the rods leave interconnected voids between them.

11. The intervertebral device of claim 9 or 10, wherein the rods form a three dimensional structure, said structure in particular being a trabecular structure as found in cancellous bone.

12. The intervertebral device of any one of claims 9 to 11, wherein the rods have a metallic surface and/or are made from a metal or an alloy.

13. The intervertebral device of claim 12, wherein the metallic surface is made from tantalum, niobium, titanium or an alloy of any two or more thereof.

14. The intervertebral device of any one of claims 9 to 13, wherein the rods consist of metal coated pyrolized polymeric material.

15. A method for stabilizing at least two adjacent vertebrae, comprising the steps of providing an intervertebral device (1) according to any one of claims 1 to 14 and impeding relative movement between the vertebrae by placing the intervertebral device (1) between two spinous processes (SP1, SP2) of the vertebrae.
